(19) Europäisches Patentamt
European Patent Office
Office européen des brevets

(11) Veröffentlichungsnummer: **0 101 925**
**B1**

(12) **EUROPÄISCHE PATENTSCHRIFT**

(45) Veröffentlichungstag der Patentschrift: **26.11.86**

(21) Anmeldenummer: **83107306.9**

(22) Anmeldetag: **26.07.83**

(51) Int. Cl.⁴: **C 07 D 249/08,** C 07 D 233/60, C 07 D 233/56, A 01 N 43/653, A 01 N 43/50

(54) **Substituierte Azolylvinylketone und -carbinole.**

(30) Priorität: **05.08.82 DE 3229274**

(43) Veröffentlichungstag der Anmeldung: **07.03.84 Patentblatt 84/10**

(45) Bekanntmachung des Hinweises auf die Patenterteilung: **26.11.86 Patentblatt 86/48**

(84) Benannte Vertragsstaaten: **AT BE CH DE FR GB IT LI NL SE**

(56) Entgegenhaltungen:
**EP-A-0 015 387**
**EP-A-0 032 239**
**EP-A-0 040 367**
**EP-A-0 040 368**
**EP-A-0 044 425**
**EP-A-0 044 993**
**EP-A-0 053 311**
**EP-A-0 057 357**
**DE-A-3 028 337**

(73) Patentinhaber: **BAYER AG, Konzernverwaltung RP Patentabteilung, D-5090 Leverkusen 1 Bayerwerk (DE)**

(72) Erfinder: **Elbe, Hans- Ludwig, Dr., Dasnöckel 59, D-5600 Wuppertal 11 (DE)**
Erfinder: **Reiser, Wolf, Dr., Kiebitzweg 12 a, D-5600 Wuppertal 1 (DE)**
Erfinder: **Regel, Erik, Untere Bergerheide 26, D-5600 Wuppertal 1 (DE)**
Erfinder: **Büchel, Karl Heinz, Prof. Dr., Dabringhausener Strasse 42, D-5093 Burscheid (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen (DE)**
Erfinder: **Lürssen, Klaus, Dr., August- Kierspel-Strasse 89, D-5060 Bergisch- Gladbach 2 (DE)**
Erfinder: **Frohberger, Paul- Ernst, Dr., Willi Baumeister- Strasse 5, D-5090 Leverkusen 1 (DE)**

**EP 0 101 925 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue substituierte Azolylvinyl-ketone und -carbinole, mehrere Verfahren zu ihrer Herstellung sowie ihre Verwendung als Fungizide und Pflanzenwachstumsregulatoren.

Es ist bereits bekannt geworden, daß bestimmte 1-Vinyltri-azolyl-(halogen)-tert.-butyl-ketone und -carbinole gute fungizide Eigenschaften besitzen (vergleiche DE- A 29 06 061, EP-A 0 015 387, DE-A 29 38 422, DE-A 30 19 046, EP-A 0 040 368, EP-A 0 032 239, EP-A 0 040 367, EP-A 0 044 425, EP-A 0 053 311, EP-A 0 057 357, DE-A 30 28 337, DE-A 28 38 847 sowie die Japanische Patentanmeldung J5 31 30 661). Die Wirkung dieser Verbindungen ist jedoch, insbesondere bei niedrigen Aufwandmengen und -konzentrationen, nicht immer voll befriedigend.

Ferner sind aus der EP-A 0 044 993 fungizid und pflanzenwuchsregulierend wirksame Vinyl-triazol-Derivate bekannt, in denen ein Kohlenstoffatom der Vinyl-Gruppe mit einem Wasserstoffatom und einem gegebenenfalls substituierten Phenyl-Rest verknüpft ist, und das andere Kohlenstoffatom der Vinyl-Gruppe neben dem Triazolyl-Rest noch einen über -CH (OH) -C $(Alkyl)_2$ - gebundenen, gegebenenfalls substituierten Phenyl-Rest trägt. Entsprechende Verbindungen, in denen dasjenige Kohlenstoffatom der Vinyl-Gruppe, das durch Wasserstoff substituiert ist, mit anderen Resten als einem gegebenenfalls substituierten Phenyl-Rest verbunden ist, werden jedoch nicht beschrieben. Außerdem werden auch nicht solche Verbindungen offenbart, in denen der über -CH (OH) - C $(Alkyl)_2$ - gebundene Rest andere Bedeutungen hat als Alkyl mit mehr als einem Kohlenstoffatom, gegebenenfalls substituiertes Phenyl oder Alkoxycarbonyl, Auch die Wirkung derjenigen Stoffe, die aus der EP-A 0 044 993 bekannt sind, läßt bei geringen Dosierungen in manchen Fällen zu wünschen übrig.

Es wurden neue substituierte Azolylvinyl-ketone und -carbinole der Formel

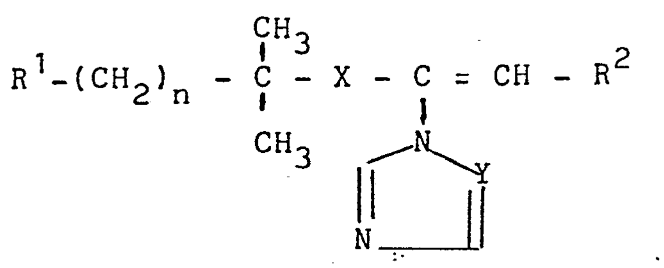

(I)

in welcher

$R^1$ für gegebenenfalls einfach bis dreifach, gleich oder verschieden substituiertes Phenyl steht, wobei als Substituenten Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino und gegebenenfalls durch Fluor, Chlor und Methyl substituiertes Phenyl oder Phenoxy zu nennen sind; ferner für Vinyl, Propargyl, Cyano, sowie die Gruppierung - Z-$R^3$ steht;

$R^3$ für

jeweils gegebenenfalls einfach bis dreifach, gleich oder verschieden substituiertes Phenyl oder Benzyl steht, wobei jeweils als Phenylsubstituenten die bei $R^1$ bereits oben genannten Phenylsubstituenten infrage kommen;

Z für O, S, SO oder $SO_2$ steht,

n für die Zahlen 0 bis 2 steht,

$R^2$ für geradkettiges oder verzweigtes Alkyl mit 1 bis 8 Kohlenstoffatomen und geradkettiges oder verzweigtes Alkenyl und Alkinyl mit jeweils 2 bis 4 Kohlenstoffatomen steht; ferner für jeweils gegebenenfalls einfach bis dreifach, gleich oder verschieden substituiertes Phenyl und Phenylalkyl mit 1 bis 2 Kohlenstoffatomen im Alkylteil steht, wobei jeweils als Phenylsubstituenten die bie $R^1$ bereits genannten Phenylsubstituenten infrage kommen; weiterhin für gegebenenfalls einfach bis dreifach, gleich oder verschieden durch Methyl, Ethyl, Isopropyl, Fluor oder Chlor substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cycloheptylmethyl, Cycloalkenyl mit 3 bis 7 Kohlenstoffatomen, Cycloalkenylmethyl mit 3 bis 7 Kohlenstoffatomen im Cycloalkenylteil, Bicycloalkyl mit 5 bis 12 Kohlenstoffatomen oder Bicycloalkenyl mit 5 bis 12 Kohlenstoffatomen steht; wobei jedoch nicht gleichzeitig $R^2$ für gegebenenfalls substituiertes Phenyl und $R^1$ für gegebenenfalls substituiertes Phenyl stehen dürfen;

X für die CO- oder CH(OH)-Gruppe steht und

Y für ein Stickstoffatom oder die CH-Gruppe steht,

sowie deren Säureadditions-Salze und Metallsalz-Komplexe gefunden.

Die erfindungsgemäßen Verbindungen der Formel (I) kommen in den geometrischen Isomeren E (trans) und Z (cis) vor. Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet von zusammen) vor, stehen sie auf entgegengesetzter Seite, liegt die Konfiguration E (abgeleitet von entgegen) vor.

Außerdem besitzen die erfindungsgemäßen Verbindungen der Formel (I) für X = CH(OH) zwei asymmetrische Kohlenstoffatome; sie können dann in den beiden geometrischen Isomeren (threo- und erythro-Form) vorliegen, die in unterschiedlichen Mengenverhältnissen anfallen können. In beiden Fällen liegen sie als optische Isomere vor.

Weiterhin wurde gefunden, daß man die substituierten Azolylvinyl-ketone und -carbinole der Formel (I) sowie deren Säureadditions-Salze und Metallsalz-Komplexe erhält, wenn man

a) Ketoenamine der Formel

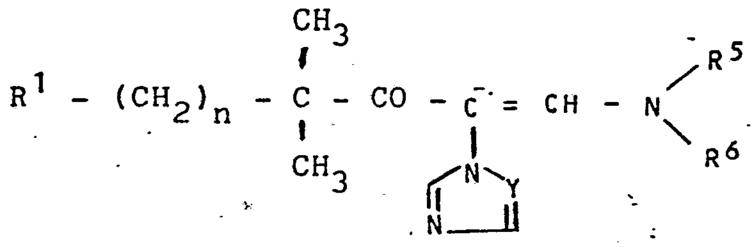

(II)

in welcher

$R^1$, Y und n die oben angegebene Bedeutung haben und

$R^5$ und $R^6$ gleich oder verschieden sind und für Alkyl mit 1 bis 4 Kohlenstoffatomen stehen; oder gemeinsam mit dem N-Atom, an das sie gebunden sind, für jeweils gegebenenfalls einfach oder dreifach durch Alkyl mit 1 bis 4 Kohlenstoffatomen substituiertes Piperidinyl, Pyrrolidinyl und Morpholinyl stehen,

mit magnesium-organischen Verbindungen der Formel

$Hal - Mg - R^2$ (III)

in welcher

$R^2$ die oben angegebene Bedeutung hat und

Hal für Halogen steht,

in Gegenwart eines Lösungsmittels und gegebenenfalls in Gegenwart eines Inertgases umsetzt, oder

b) Azolylketone der Formel

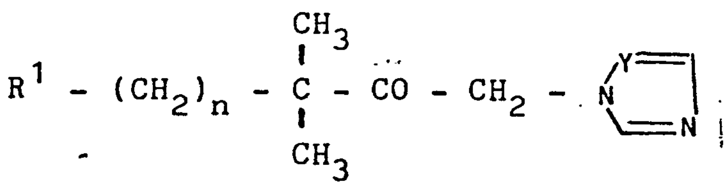

(IV).

in welcher

$R^1$, Y und n die oben angegebene Bedeutung haben,

mit Aldehyden der Formel

$O = CH - R^2$ (V)

in welcher

$R^2$ die oben angegebene Bedeutung hat,

in Gegenwart eines Lösungsmittels und in Gegenwart eines Katalysators umsetzt, und

c) gegebenenfalls die nach den Verfahren (a) und (b) erhaltenen erfindungsgemäßen substituierten Azolylvinyl-ketone der Formel

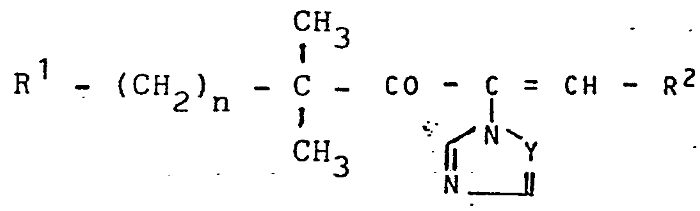

(Ia)

$R^1$, $R^2$, Y und n die oben angegebene Bedeutung haben,
in allgemein üblicher Weise reduziert
und gegebenenfalls an die so erhaltenen Verbindungen der Formel (I) noch eine Säure oder ein Metallsalz addiert.

Schließlich wurde gefunden, daß die neuen substituierten Azolylvinyl-ketone und -carbinole der Formel (I) sowie deren Säureadditions-Salze und Metallsalz-Komplexe starke fungizide und starke pflanzenwachstumsregulierende Eigenschaften aufweisen.

Außerdem sind die neuen substituierten Azolylvinylketone und -carbinole der Formel (I) interessante Zwischenprodukte zur Herstellung von weiteren Pflanzenschutzmitteln. Bei den Keto- Derivaten kann die Ketogruppe zu einer -CH(OH)-Gruppe bzw. einer -CH(OH)-Gruppe reduziert werden. Ferner können durch entsprechende Umsetzung funktionelle Derivate der Ketogruppe erhalten werden, wie z.B. Oxime und Oximether, Hydrazone und Ketale. Die Carbinol-Derivate können an der Hydroxygruppe in üblicher Weise in die entsprechenden Ether überführt werden. Weiterhin können durch Umsetzung mit z.B. Acylhalogeniden oder Carbamoylchloriden in prinzipiell bekannter Weise Acyl- oder Carbamoyl-Derivate der Verbindungen der Formel (I) erhalten werden.

Ueberraschenderweise besitzen die erfindungsgemäßen Verbindungen bessere fungizide Wirkung als die aus dem Stand der Technik bekannten 1-Vinyltriazolyl-(halogen)tert.-butyl-ketone und -carbinole, welche chemisch und biologisch naheliegende Verbindungen sind. Außerdem zeigen die erfindungsgemäßen Verbindungen gute pflanzenwachstumsregulierende Eigenschaften. Die erfindungsgemäßen Wirkstoffe stellen somit eine Bereicherung der Technik dar.

Die erfindungsgemäßen substituierten Azolylvinyl-ketone und -carbinole sind durch die Formel (I) allgemein definiert. Sie unterscheiden sich bezüglich der Substituenten-Definitionen in charakteristischer Weise von den aus dem Stand der Technik bekannten Vinyl-azolyl-Derivaten (Vgl. zum Beispiel EP-A 0 044 993).

Bevorzugte erfindungsgemäße Verbindungen sind Additionsprodukte aus Säuren und denjenigen Azolylvinylketonen und -carbinolen der Formel (I), in denen $R^1$,$R^2$, X, Y und n die Bedeutungen haben, die bereits für diese Reste bzw. diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäureund 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolylvinyl-ketonen undcarbinolen der Formel (I), in denen $R^1$, $R^2$, X, Y und n die Bedeutungen haben, die bereits für diese Reste bzw. diesen Index genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäuren und die Bromwasserstoffsäure, ferner Phosphorsäure, Salpetersäure und Schwefelsäure.

Verwendet man beispielsweise 1-(4-Chlorphenyl)-2,2-dimethyl-5-dimethylamino-4-(1,2,4-triazol-1-yl)-4-penten-3-on und n-Propylmagnesiumbromid als Ausgangsstoffe, so kann der Reaktionsablauf durch das folgende Formelschema wiedergegeben werden (Verfahren a):

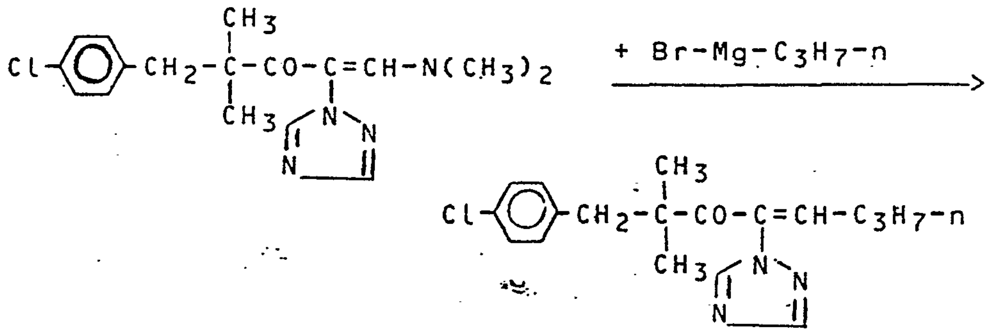


Verwendet man beispielsweise 1-(4-Chlorphenyl)-2,2-dimethyl-4-(1,2,4-triazol-1-yl)-3-butanon und Cyclohexancarbaldehyd als Ausgangsstoffe, so kann der Reaktionsablauf durch das folgende Formelschema wiedergegeben werden (Verfahren b):

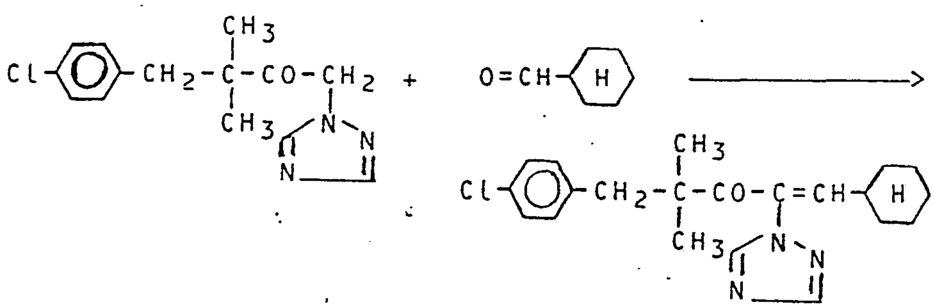

Verwendt man beispielsweise 1-(4-Chlorphenyl)-2,2-dimethyl-4-(1,2,4-triazol-1-yl)-4-octan-3-on als Ausgangsstoff und Natriumborhydrid als Reduktionsmittel, so kann der Reaktionsablauf durch das folgende Formelschema wiedergegeben werden (Verfahren c):

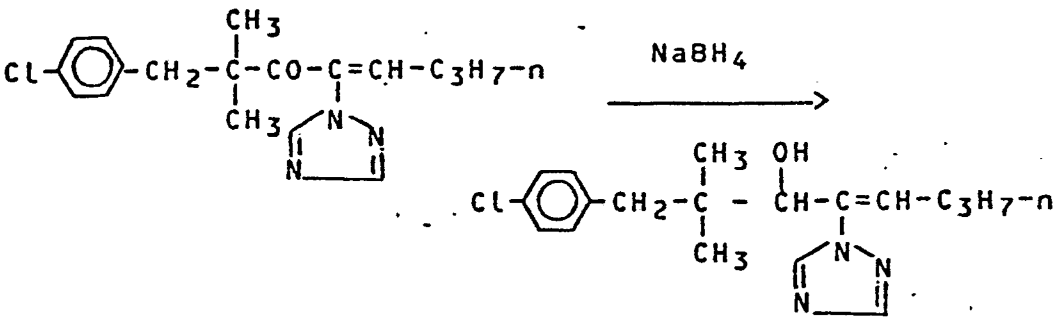


Die als Ausgangsstoffe für das erfindungsgemäße Verfahren (a) zu verwendenden Ketoenamine sind durch die Formel (II) allgemein definiert. In dieser Formel stehen $R^1$, Y und n für diejenigen Reste, die bereits im Zusammenhang mit der Beschreibung der erfindungsgemäßen Stoffe der Formel (I) für diese Substituenten bzw. diesen Index genannt wurden. $R^5$ und $R^6$ haben die in der Erfindungsdefinition angegebenen Bedeutungen.

Die Ketoenamine der Formel (II) sind noch nicht bekannt. Sie sind jedoch Gegenstand einer eigenen, parallelen Patentanmeldung (vgl. EP-A 0101 926) und werden erhalten, indem man Azolylketone der Formel

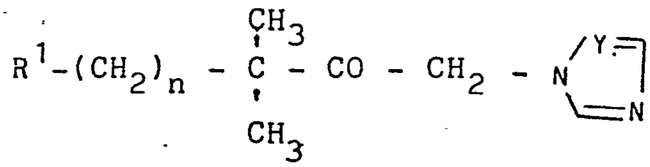

(IV)

in welcher
$R^1$, Y und n die oben angegebene Bedeutung haben, mit Amidacetalen bzw. Aminalestern der Formeln

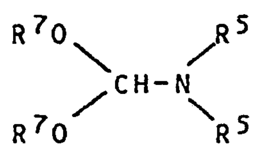

(VI a)

bzw.

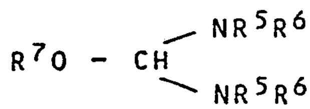

(VIb)

in welcher
$R^5$ und $R^6$ die oben angegebene Bedeutung haben
und
$R^7$ für Alkyl mit 1 bis 4 Kohlenstoffatomen steht,
in an sich bekannter Art und Weise in Gegenwart eines inerten organischen Lösungsmittels, wie beispielsweise eines aromatischen Kohlenwasserstoffes und wie insbesondere eines im Ueberschuß eingesetzten Amidacetals bzw. Aminalesters der Formel (VIa) bzw. (VIb) in der Siedehitze umsetzt (vergleiche hierzu auch Chem.Ber. 101, 41-50 (1968); J.Org.Chem. 43, 4248-50 (1978) sowie die Herstellungsbeispiele).

Die Azolylketone der Formel (IV) sind weitgehend bekannt (vergleiche hierzu DE-A 24 31 407, DE-A 29 06 061, DE-A 30 28 330, DE-A 30 48 266, EP-A 0 081 675 und EP-A 0 080 096). Sie können nach üblichen Methoden hergestellt werden, indem man die entsprechenden Halogenketone in Gegenwart eines Säurebinders mit 1,2,4-Triazol oder Imidazol umsetzt.

Die Amidacetale bzw. Aminalester der Formeln (VIa) bzw. (VIb) sind allgemein bekannte Verbindungen der organischen Chemie (vergleiche z.B. Chem. Ber. 101, 41 -50 (1968) und J.Org. Chem. 43, 4248-50 (1978)); bzw. können sie nach den dort angegebenen Verfahren erhalten werden.

Die außerdem für das erfindungsgemäße Verfahren (a) als Ausgangsstoffe zu verwendenden magnesium-organischen Verbindungen sind durch die Formel (III) allgemein definiert. In dieser Formel steht $R^2$ für diejenigen Reste, die im Zusammenhang mit der Beschreibung der erfindungsgemäßen Stoffe der Formel (I) bereits für diesen Substituenten genannt wurden. Hal steht vorzugsweise für Chlor oder Brom.

Die magnesium-organischen Verbindungen der Formel (III) sind allgemein bekannte Verbindungen der organischen Chemie.

Die als Ausgangsstoffe für das erfindungsgemäße Verfahren (b) zu verwendenden Azolylketone sind durch die Formel (IV) allgemein definiert. In dieser Formel stehen $R^1$, Y und n für diejenigen Reste, die im Zusammenhang mit der Beschreibung der erfindungsgemäßen Stoffe der Formel (I) bereits für diese Substituenten bzw. diesen Index genannt wurden.

Die außerdem für das erfindungsgemäße Verfahren (b) als Ausgangsstoffe zu verwendenden Aldehyde sind durch die Formel (V) allgemein definiert. In dieser Formel steht $R^2$ für diejenigen Reste, die im Zusammenhang mit der Beschreibung der erfindungsgemäßen Stoffe der Formel (I) bereits für diesen Substituenten genannt

wurden.

Die Aldehyde der Formel (V) sind allgemein bekannte Verbindungen der organischen Chemie.

Die als Ausgangsstoffe für das erfindungsgemäße Verfahren (c) zu verwendenden Azolylvinyl-ketone sind erfindungsgemäße Stoffe.

Als Lösungsmittel kommen für die erfindungsgemäße Umsetzung gemäß Verfahren (a) vorzugsweise inerte organische Lösungsmittel in reiner Form oder im Gemisch infrage. Hierzu gehören vorzugsweise Ether, wie Diethylether, Methylethylether, Tetrahydrofuran oder Dioxan, aliphatische und aromatische Kohlenwasserstoffe, wie insbesondere Benzol, Toluol oder Xylol, sowie Hexamethylphosphorsäuretriamid.

Die Reaktionstemperaturen können bei der Durchführung des erfindungsgemäßen Verfahrens (a) in einem größeren Bereich variiert werden. Im allgemeinen arbeitet man zwischen -50 und +100°C, vorzugsweise zwischen -20 und +120°C.

Die erfindungsgemäße Umsetzung gemäß Verfahren (a) kann in Gegenwart eines Inertgases, wie z.B. Stickstoff oder Helium, durchgeführt werden.

Bei der Durchführung des erfindungsgemäßen Verfahrens (a) setzt man auf 1 Mol Ketoenamin der Formel (II) vorzugsweise 1 bis 1,5 Mol an magnesium-organischen Verbindungen der Formel (III) ein. Die Isolierung der Verbindungen der Formel (I) erfolgt in üblicher Weise.

Als Lösungsmittel kommen für das erfindungsgemäße Verfahren (b) vorzugsweise inerte organische Lösungsmittel infrage. Hierzu gehören vorzugsweise Alkohole, wie Methanol und Ethanol; Ether, wie Tetrahydrofuran und Dioxan; aliphatische und cycloaliphatische Kohlenwasserstoffe, wie Hexan und Cyclohexan; aromatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol und Dichlorbenzol.

Das erfindungsgemäße Verfahren (b) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen, wie Pyridin und Piperidin; sowie insbesondere Piperidinacetat.

Die Reaktionstemperaturen können bei der Durchführung des erfindungsgemäßen Verfahrens (b) in einen größeren Bereich variiert werden. In allgemeinen arbeitet man zwischen 20 und 160°C, vorzugsweise bei der Siedetemperatur des jeweiligen Lösungsmittels.

Bei der Durchführung des erfindungsgemäßen Verfahrens (b) setzt man auf 1 Mol Triazol-keton der Formel (IV) 1 bis 1,5 Mol Aldehyd der Formel (V) und katalytische bis 0,2 molare Mengen an Katalysator ein. Die Isolierung der Verbindungen der Formel (I) erfolgt in üblicher Weise.

Die erfindungsgemäße Reduktion gemäß Verfahren (c) erfolgt in üblicher Art und Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für diese erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (Ia) etwa 1 Reaktionsäquivalent eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für diese erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstehende Aluminiumverbindung mit verdünnter Schwefelsäure oder Natronlauge zersetzt. Die weitere Aufarbeitung erfolgt in üblicher Weise.

Zur Herstellung von Säureadditions-Salzen der Azolylvinyl-ketone und -carbinole der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen von Azolyl-vinylketonen und -carbinolen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalz-Komplexe in bekannter Weise, z.B. durch Abfiltrieren, isolieren

und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt ein Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten eingesetzt werden; so z.B. gegen den Erreger des Gerstenmehltaus (Erysiphe graminis), den Erreger des Braunrostes am Weizen (Puccinia recondita), den Erreger der Streifenkrankheit der Gerste (Cochliobolus sativus), den Erreger der Braunfleckenkrankheit am Weizen (Leptoshaeria nodorum) oder den Erreger der Blattfleckenkrankheit an Getreide (Pyrenophora teres); ferner zur Bekämpfung von Venturia -Arten, wie gegen den Erreger des Apfelschorfes (Venturia inaequalis) sowie von Reiskrankheiten, wie Pyricularia und Pellicularia.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige wirkungen auf Pflanzen ausüber kann. Die Wirkungen der Stoffe hängen im wesentlichen ab von dem Zeitpunkt der Anmeldung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen; an Straßenrändern, auf Flughäfen oder in Obstanlagen reduziert werden. Von Bedeutung ist auch die Hemmung des Wuchses von krautigen und holzigen Pflanzen an Straßenrädern und in der Nähe von Pipelines oder Überlandleitungen oder ganz allgemein in Bereichen, in denen ein starker Zuwachs von Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können wachstumsregulatoren bei Getreide eine Halmverstärkung hervorufen, die ebenfalls dem Lagern entgegenwirkt. Die Anwendung von Wachstumsregulatoren zur Halmverkürzung und Halmverstärkung erlaubt es, höhere Düngermangen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt aich häufig auch eine Förderung des vegetativen Washstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt verden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß

ein Entblättern der Pflanzen zu einem gewünschten Zeitpunkt erreicht vird. Eine derartige Entlaubung spielt bei der mechanischen Beerntung der Baumwolle eine große Rolle ist aber auch in anderen Kulturen die z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Kemmung won Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch vermischen der Wirkstoffe mit Streckmitteln, also flüssigen Lösungsmitteln, unter Druck stehenden verflüssigten Gasen und / oder festen Trägerstoffen, gegebenenfalls unter Verwendung won oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatisshe Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel vie Carboxymethylcellulose, natürliche und synthetische pulverige, körnige oder latexförmige Polymere werwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe, wie Salze von Eisen, Mangan, Bor, Kupfer, Kobalt, Molybdän und Zink werwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume,

Suspensionen, Spritzpulwer, Pasten, lösliche Pulwer, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50g je kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,2 %, am Wirkungsort erforderlich.

Die Herstellung und die Verwendung der erfindungsgemäßen Stoffe werden durch die folgenden Beispiele veranschaulicht.

**Herstellungsbeispiele**

**Beispiel 1**

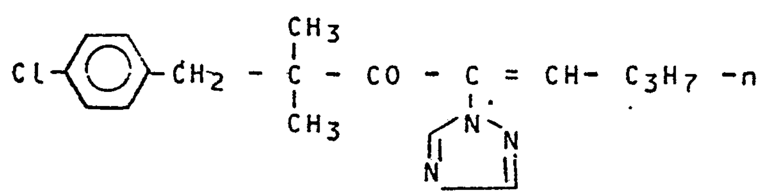

(Verfahren a)

49,9g (0,15 Mol) 1-(4-Chlorphenyl)-2,2-dimethyl-5-dimethylamino-4-(1,2,4-triazol-1-yl)-4-penten-3-on werden in 750 ml Ether gelöst und bei -20°C tropfenweise mit einer Lösung von 33,9g (0,23-Mol) n-Propylmagnesiumbromid in 100 ml Ether versetzt. Man läßt 1,5 Stunden nachrühren, wobei sich das Reaktionsgemisch auf Raumtemperatur erwärmt. Mit verdünnter Salzsäure wird das Reaktionsgemisch auf einen pH-Wert von 7 bis 8 eingestellt. Danach wird die organische Phase abgetrennt, mit Wasser gewaschen, über Natriumsulfat getrocknet und eingeengt. Der Rückstand wird säulenchromatographisch (Kieselgel; Essigester/Cyclohexan = 3:1) gereinigt. Man erhält 30,1 g (60,5 % der Theorie) 1-(4-Chlorphenyl)-2,2-dimethyl-4-(1,2,4-triazol-1-yl)-4-octen-3-on vom Brechungsindex $n^{20}_D$-1,5429.

**Herstellung des Ausgangsproduktes**

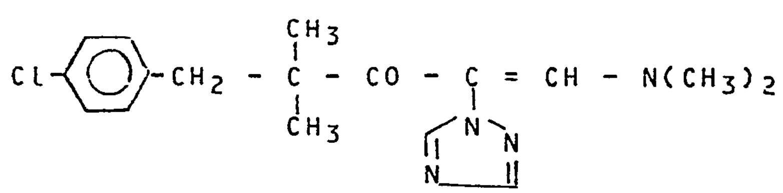

50g (0,18 Mol) 1-(4-Chlorphenyl)-2,2-dimethyl-4-(1,2,4-triazol-1-yl)-3-butanon werden mit 23,6g (0,138 Mol) Dimethylformamid-dimethylacetal 8 Stunden unter Rückfluß erhitzt. Zur Isolierung des Endproduktes wird das Reaktionsgemisch im Vakuum eingeengt. Man erhält 56,5 g-(94,4 % der Theorie) 1-(4-Chlorphenyl)-2,2-dimethyl-5-dimethyl-amino-4-(1,2,4-triazol-1-yl)-4-penten-3-on vom Brechungsindex $n_D^{20}$ = 1,5797.

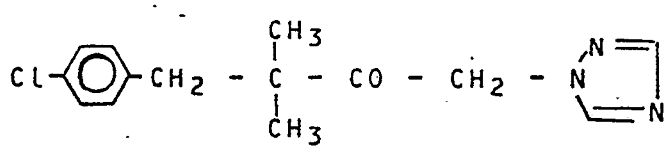

37g (0,13 Mol) 4-Brom-1-(4-chlorphenyl)-2,2-dimethyl-3-butanon, 13,3g (0,019 Mol) 1,2,4-Triazol und 53,8g (0,39 Mol) Kaliumcarbonat werden in 300 ml Aceton 8 Stunden unter Rückfluß erhitzt. Man läßt abkühlen, saugt vom anorganischen Rückstand ab und engt das Filtrat ein. Der Rückstand wird in Chloroform aufgenommen, mit Wasser gewaschen, über Natriumsulfat getrocknet und eingeengt. Der Rückstand wird mit Diethylether verrührt, abgesaugt und bei 50°C im Vakuum getrocknet. Man erhält 18,8g (52 % der Theorie) 1-(4-Chlorphenyl)-2,2-dimethyl-4-(1,2,4-triazol-1-yl)-3-butanon vom Schmelzpunkt 127°C.

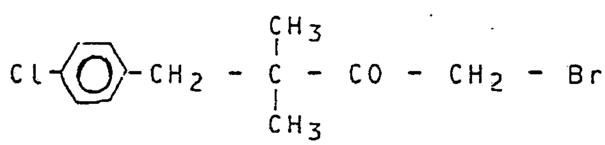

130g (0,62 Mol) 1-(4-Chlorphenyl)-2,2-dimethyl-3-butanon in 1000 ml Chloroform werden bei Raumtemperatur tropfenweise mit 98,8g (0,62 Mol) Brom versetzt. Man läßt das Reaktionsgemisch 1 Stunde nachrühren und engt anschließend ein. Man erhält 174,7g (97,3 % der Theorie) 4-Brom-1-(4-chlorphenyl)-2,2-dimethyl-3-butanon vom Brechungsindex $n_D^{20}$ = 1,5570.

**Beispiel 2**

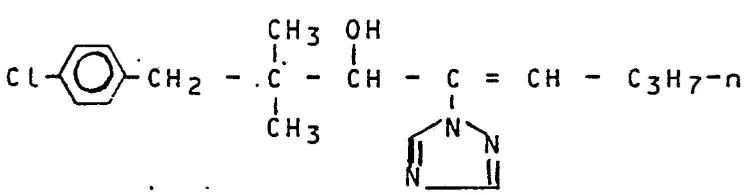

(Verfahren c)

7g (0,021 Mol) 1-(4-Chlorphenyl)-2,2-dimethyl-4-(1,2,4-triazol-1-yl)-4-octen-3-on (Beispiel 1) werden in 100 ml Methanol gelöst und bei -10°C tropfenweise mit einer Lösung von 0,21g (0,006 Mol) Natriumborhydrid in 5 ml Eiswasser versetzt. Man läßt 1,5 Stunden bei 0°C nachrühren und stellt dann das Reaktionsgemisch mit verdünnter Salzsäure auf einen pH-Wert von 6 bis 7. Das Reaktionsgemisch wird durch Abdestillieren des Lösungsmittels im Vakuum eingeengt. Der Rückstand wird in Chloroform aufgenommen, mit Wasser gewaschen, über Natriumsulfat getrocknet und eingeengt. Man erhält 6,5g (92,9 % der Theorie) 1-(4-Chlorphenyl)-2,2-dimethyl-4-(1,2,4-triazol-1-yl)-4-octen-3-ol vom Brechungsindex $n^{20}_D$ = 1,5383.

**Beispiel 3**

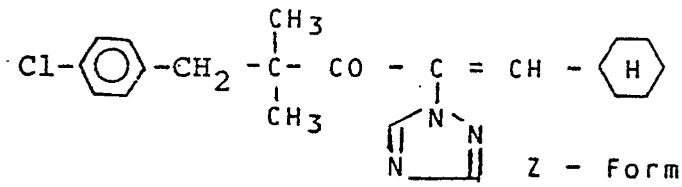

Z - Form

8,2g (0,03 Mol) 1-(4-Chlorphenyl)-2,2-dimethyl-4-(1,2,4-triazol-1-yl)-3-butanon, 3,4g (0,03 Mol) Cyclohexancarbaldehyd, 1,1g (0,018 Mol) Essigsäure und 0,8 g (0,009 Mol) Piperidin werden in 60 ml Toluol gelöst und am Wasserabscheider 8 Stunden unter Rückfluß erhitzt. Die abgekühlte Reaktionslösung wird mit Wasser gewaschen, die organische Phase mit Natriumhydrogensulfitlösung extrahiert, der ausgefallene Niederschlag abgenutscht und die Toluolphase mit Natriumhydrogencarbonat-Lösung gewaschen. Nach dem Trocknen über Natriumsulfat wird das Lösungsmittel am Rotationsverdampfer abgezogen und der ölige Rückstand (11,0 g) über Kieselgel mit Petrolether-Eisessig (2:1) an einer Säule chromathographiert. Die ersten Fraktionen wurden vereinigt und das Lösungsmittelgemisch am Rotationsverdampfer abgezogen.

Man erhält 3,0g (26,9 % der Theorie) 1-(4-Chlorphenyl)-5-cyclohexyl-2,2-dimethyl-4-(1,2,4-triazol-1-yl)-4-penten-3-on (Z-Form) als helles Oel.

20 MHz-NMR, $CDCl_3$ ($\delta$ ,ppm)

1.12 (s, 6H); 1.20 (m,6H); 1.70 (m, 5H); 2,90 (s,2H); 6.35 (d,1H); 7.05 (d,2H); 7.23 (d,2H); 8.00(s,1H); 8.07 (s,1H)

In entsprechender Weise und gemäß den angegebenen Verfahren werden die folgenden Verbindungen der allgemeinen Formel

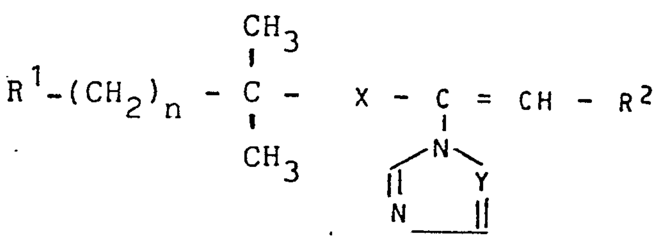

(I)

erhalten.

| Bsp. Nr. | $R^1-(CH_2)_n-\overset{CH_3}{\underset{CH_3}{C}}-$ | X | Y | $R^2$ | Schmelzpunkt (°C) bzw. $n_D^{20}$ |
|---|---|---|---|---|---|
| 4 | 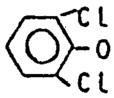 $-OCH_2-C(CH_3)_2-$ | CO | N | $-C_7H_{15}$-n | zähes Oel |
| 5 | 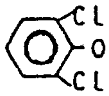 $-OCH_2-C(CH_3)_2-$ | CO | N | $-C_3H_7$-i | 36-39 |
| 6 | 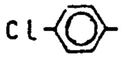 $-OCH_2-C(CH_3)_2-$ | CO | N | $-C_7H_{15}$-n | zähes Oel |
| 7 | 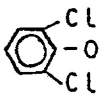 $-OCH_2-C(CH_3)_2-$ | CO | N | $-CH_3$ | 96 |
| 8 | 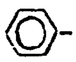 $-SCH_2-C(CH_3)_2-$ | CO | N | $-CH_2-$ 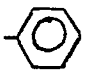 | 1,5703 |
| 9 | 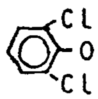 $-OCH_2-C(CH_3)_2-$ | CO | N | $-CH_2-$ 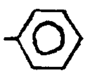 | zähes Oel |
| 10 | 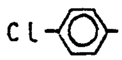 $-OCH_2-C(CH_3)_2-$ | CO | N | $-C_4H_9$-n | zähes Oel |
| 11 | 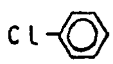 $OCH_2-C(CH_3)_2-$ | CO | N | $-CH_2-$ 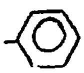 | 124 |
| 12 | 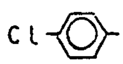 $-OCH_2-C(CH_3)_2-$ | CO | N | $-CH_2-$ 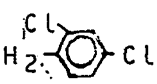 | zähes Oel |
| 13 | 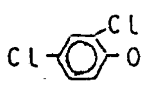 $-OCH_2-C(CH_3)_2-$ | CO | N | $-CH_2-$ 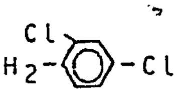 | 110 |
| 14 | 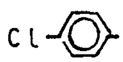 $-OCH_2-C(CH_3)_2-$ | CO | N | $-CH_2-$ 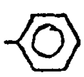 | 120 |
| 15 | 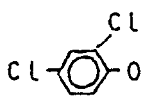 $-O-CH_2-C(CH_3)_2-$ | CO | N | $-CH_2-$ 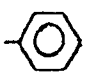 | 116 |
| 16 | 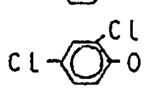 $-OCH_2-C(CH_3)_2-$ | CO | N | $-C(CH_3)_3$ | 1,5401 |
| 17 | 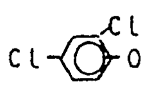 $-OCH_2-C(CH_3)_2-$ | CO | N | $-C_4H_9$-n | 1,5419 |
| 18 | 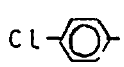 $-SCH_2-C(CH_3)_2-$ | CO | N | $-C_4H_9$-n | zähes Oel |
| 19 | 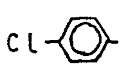 $-S-CH_2-C(CH_3)_2-$ | CO | N | $-CH_2-$ 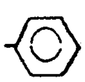 | 136-28 |
| 20 | 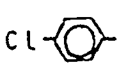 $-OCH_2-C(CH_3)_2-$ | CO | N | $-CH(CH_3)C_2H_5$ | zähes Oel |

| Bsp. Nr. | $R^1-(CH_2)_n-\overset{CH_3}{\underset{CH_3}{C}}-$ | X | Y | $R^2$ | Schmelzpunkt (°C) bzw. $n_D^{20}$ |
|---|---|---|---|---|---|
| 21 | 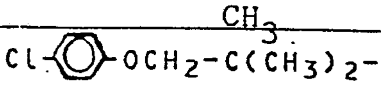 | CO | N | $-C_3H_7$-i | zähes Oel |
| 22 | 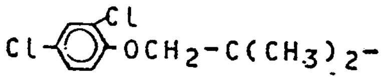 | CO | N | $-C_2H_5$ | zähes Oel |
| 23 | 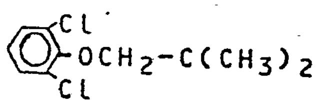 | CO | N | 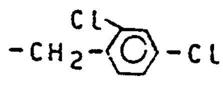 | zähes Oel |
| 24 | 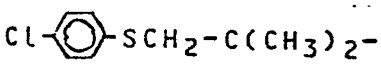 | CO | N | $-C_3H_7$-n | 1,5679 |
| 25 | 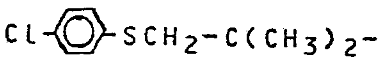 | CO | N | $-C_2H_5$ | 1,5662 |
| 26 | 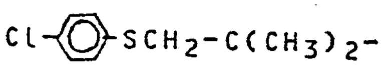 | CO | N | $-CH_3$ | 1,5749 |
| 27 | 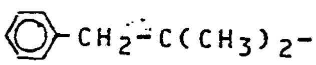 | CO | N | 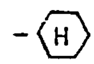 | 76-80 (Z-Form) |
| 28 | 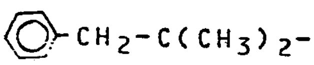 | CO | N | 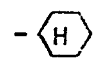 | 63-70 |
| 29 | 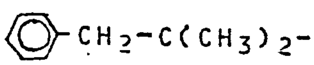 | CO | N | 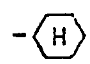 | 87-97 (E-Form) |
| 30 | 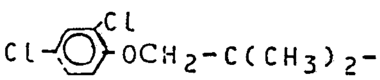 | CO | N | 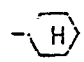 | zähes Oel (Z-Form) |
| 31 | 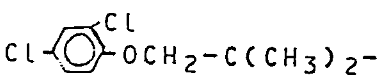 | CO | N | 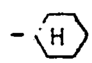 | zähes Oel |
| 32 | 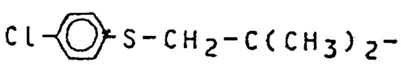 | CO | N | 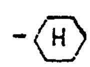 | zähes Oel (Z-Form) |
| 33 | 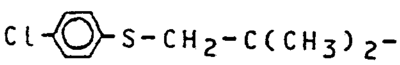 | CO | N | 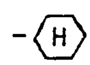 | zähes Oel |
| 34 | 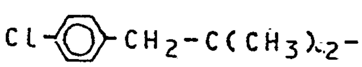 | CO | N | 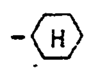 | zähes Oel |
| 35 | 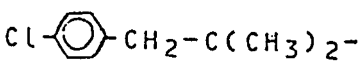 | CO | N | 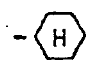 | zähes Oel (E-Form) |
| 36 | 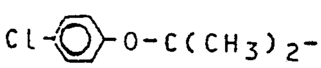 | CO | N | $-CH(CH_3)C_2H_5$ | 1,5372 |
| 37 | 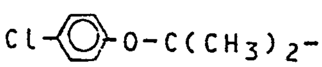 | CO | N | $-C_3H_7$-n | 1,5421 |
| 38 | 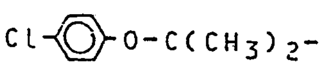 | CO | N | $-C_2H_5$ | zähes Oel |
| 39 | 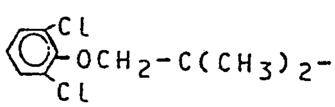 | CH(OH) | N | $-C_3H_7$-i | zähes Oel |
| 40 | 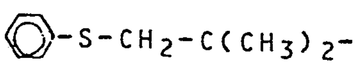 | CH(OH) | N | $-C_4H_9$-n | zähes Oel |

| Bsp. Nr | CH₃<br>$R^1-(CH_2)_n-C-$<br>CH₃ | X | Y | $R^2$ | Schmelzpunkt (°C) bzw. $n_D^{20}$ |
|---|---|---|---|---|---|
| 41 | 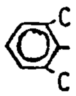 $OCH_2-C(CH_3)_2-$ | CH(OH) | N | $-CH_2-$ 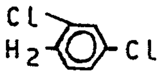 | zähes Oel |
| 42 | 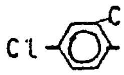 $O-CH_2-C(CH_3)_2-$ | CH(OH) | N | $-C_4H_9-n$ | 1,5333 |
| 43 | 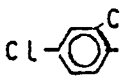 $O-CH_2-C(CH_3)_2-$ | CH(OH) | N | $-C_2H_5$ | 30 |
| 44 | 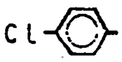 $O-CH_2-C(CH_3)_2-$ | CH(OH) | N | $-CH(CH_3)C_2H_5$ | zähes Oel |
| 45 | 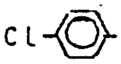 $O-CH_2-C(CH_3)_2-$ | CO | N | $-C_3H_7-i$ | 30 |
| 46 | 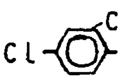 $OCH_2-C(CH_3)_2-$ | CH(OH) | N | $-CH_2-$ 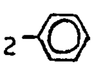 | 54-56 |
| 47 | 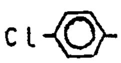 $SCH_2-C(CH_3)_2-$ | CH(OH) | N | $-CH_2-$ 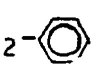 | 120-24 |
| 48 | 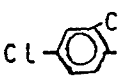 $OCH_2-C(CH_3)_2-$ | CH(OH) | N | $-CH_2-$ 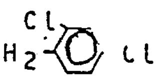 | zähes Oel |
| 49 | 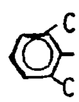 $OCH_2-C(CH_3)_2-$ | CH(OH) | N | $-C_4H_9-n$ | zähes Oel |
| 50 | 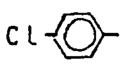 $OCH_2-C(CH_3)_2-$ | CH(OH) | N | $-C_4H_9-n$ | zähes Oel |
| 51 | 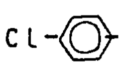 $SCH_2-C(CH_3)_2-$ | CH(OH) | N | $-C_4H_9-n$ | zähes Oel |
| 52 | 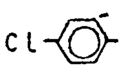 $OCH_2-C(CH_3)_2-$ | CH(OH) | N | $-CH_2-$ 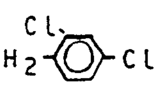 | zähes Oel |
| 53 | 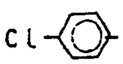 $SCH_2-C(CH_3)_2$ | CH(OH) | N | $-C_3H_7-n$ | 1,5690 |
| 54 | 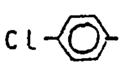 $SCH_2-C(CH_3)_2-$ | CH(OH) | N | $-CH_3$ | 1,5638 |
| 55 | 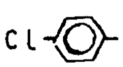 $SCH_2-C(CH_3)_2-$ | CH(OH) | N | $-C_2H_5$ | 1,5544 |
| 56 | 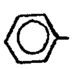 $S-CH_2-C(CH_3)_2-$ | CH(OH) | N | - 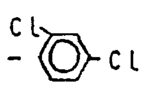 | 1,5910 (E-Form) |
| 57 | 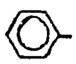 $SCH_2-C(CH_3)_2-$ | CH(OH) | N | - 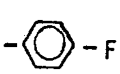 | 139 |
| 58 | 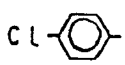 $OCH_2-C(CH_3)_2-$ | CH(OH) | N | - 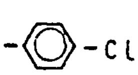 | 96(Z-Form) |
| 59 | 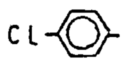 $OCH_2-C(CH_3)_2-$ | CH(OH) | N | - 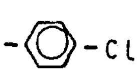 | 176(E-Form) |
| 60 | 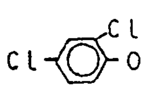 $OCH_2-C(CH_3)_2-$ | CH(OH) | N | - 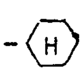 | zähes Oel (Z-Form) |

| Bsp. Nr. | $R^1-(CH_2)_n-\overset{CH_3}{\underset{CH_3}{C}}-$ | X | Y | $R^2$ | Schmelzpunkt (°C) bzw. $n_D^{20}$ |
|---|---|---|---|---|---|
| 61 | 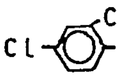$OCH_2-C(CH_3)_2-$ | CH(OH) | N | -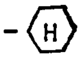 | amorph |
| 62 | 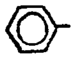$-CH_2-C(CH_3)_2-$ | CH(OH) | N | -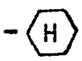 | zähes Oel (E-Form) |
| 63 | 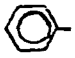$-CH_2-C(CH_3)_2-$ | CH(OH) | N | -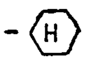 | zähes Oel (Z-Form) |
| 64 | 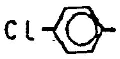$-O-C(CH_3)_2-$ | CH(OH) | N | $-C_2H_5$ | zähes Oel |
| 65 | 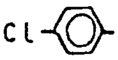$-O-C(CH_3)_2-$ | CH(OH) | N | $-C_3H_7-n$ | zähes Oel |
| 66 | 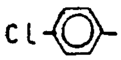$-O-C(CH_3)_2-$ | CH(OH) | N | $-CH(CH_3)C_2H_5$ | zähes Oel |
| 67 | $CH_2=CH-C(CH_3)_2-$ | CH(OH) | N | 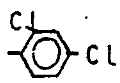 | 128 |
| 68 | 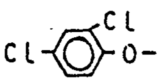$O-C(CH_3)_2-$ | CH(OH) | N | 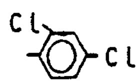 | 136 (Z-Form) |
| 69 | $NC-C(CH_3)_2-$ | CH(OH) | N | 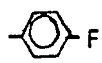 | 220 (E-Form) |
| 70 | $NC-C(CH_3)_2-$ | CH(OH) | N | 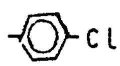 | 194 (E-Form) |
| 71 | $CH_2=CH-C(CH_3)_2-$ | CH(OH) | N | 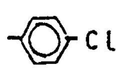 | 128 (Z-Form) |
| 72 | $CH_2=CH-C(CH_3)_2-$ | CH(OH) | N | 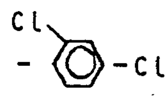 | 128 (Z-Form) |
| 73 | 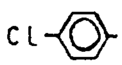$SCH_2-C(CH_3)_2-$ | CO | CH | $-CH_3$ | zähes Oel |
| 74 | 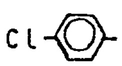$SCH_2-C(CH_3)_2-$ | CO | CH | $-C_3H_7-i$ | zähes Oel |
| 75 | 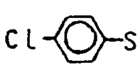$SCH_2-C(CH_3)_2-$ | CO | CH | $-C_3H_7-n$ | zähes Oel |
| 76 | 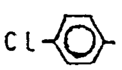$-O-C(CH_3)_2-$ | CO | CH | $-C_2H_5$ | -1,5595 |
| 77 | 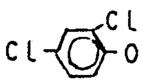$OCH_2-C(CH_3)_2-$ | CO | CH | $-C(CH_3)_2$ | 1,5463 |
| 78 | 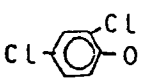$OCH_2-C(CH_3)_2-$ | CO | CH | $-CH_3$ | 1,5637 |
| 79 | 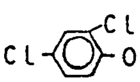$O-CH_2-C(CH_3)_2-$ | CO | CH | $-\underset{C_2H_5}{CH}-CH_3$ | 1,5420 |

| Bsp. Nr. | $R_1-(CH_2)_n-C(CH_3)_2-$ | X | Y | $R^2$ | Schmelzpunkt (°C) bzw. $n_D^{20}$ |
|---|---|---|---|---|---|
| 80 | 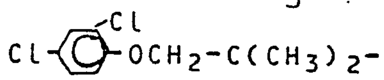 | CO | CH | $-C_7H_{15}-n$ | 1,5367 |
| 81 | 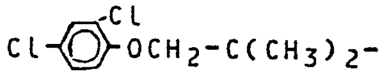 | CO | CH | $-C_3H_7-i$ | 1,5511 |
| 82 | 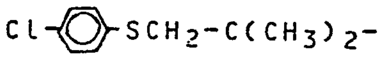 | CO | CH | $-CH(CH_3)-C_2H_5$ | 30 |
| 83 | 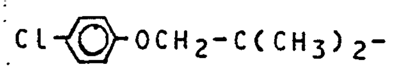 | CO | CH | $-C_3H_7-n$ | zähes Oel |
| 84 | 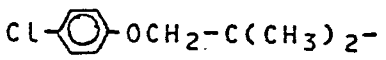 | CO | CH | $-CH(CH_3)-C_2H_5$ | zähes Oel |
| 85 | 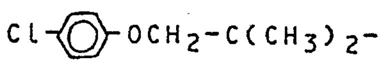 | CO | CH | $-C_2H_5$ | 53-57 |
| 86 | 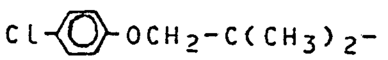 | CO | CH | $-C_4H_9-n$ | zähes Oel |
| 87 | 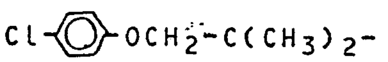 | CO | CH | $-C_3H_7-i$ | zähes Oel |
| 88 | 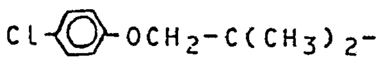 | CH(OH) | CH | $-C_3H_7-n$ | 33 |
| 89 | 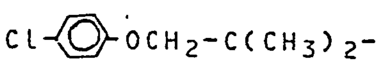 | CH(OH) | CH | $-CH(CH_3)-C_2H_5$ | 30 |
| 90 | 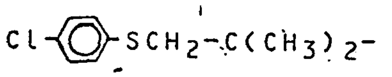 | CH(OH) | CH | $-C_3H_7-i$ | 31 |
| 91 | 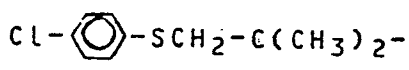 | CH(OH) | CH | $-C_3H_7-n$ | zähes Oel |
| 92 | 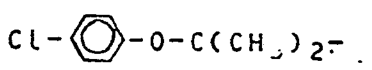 | CH(OH) | CH | $-C_2H_5$ | zähes Oel |
| 93 | 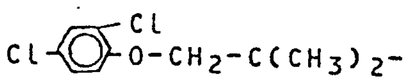 | CH(OH) | CH | $-C(CH_3)_3$ | zähes Oel |
| 94 | 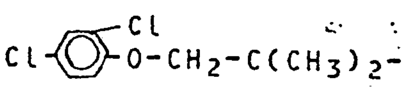 | CH(OH) | CH | $-CH(CH_3)C_2H_5$ | zähes Oel |
| 95 | 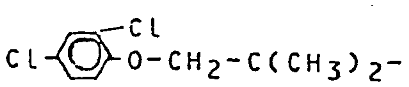 | CH(OH) | CH | $-CH_3$ | 80 |
| 96 | 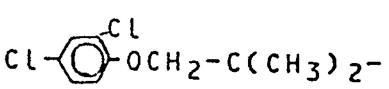 | CH(OH) | N | $-C_3H_7-i$ | zähes Oel |
| 97 | 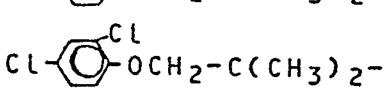 | CH(OH) | CH | $-C_7H_{15}-n$ | zähes Oel |

E- und Z-Form: die beiden möglichen geometrischen Isomeren

**Verwendungsbeispiele**

In den nachfolgenden Fungizid-Beispielen werden die nachstehend angegebenen Verbindungen als Vergleichssubstanzen eingesetzt:

(A) 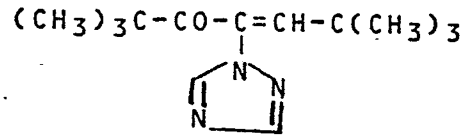

(B) 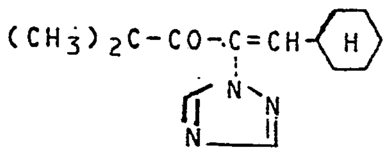

(C) 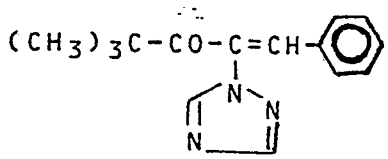

(D) 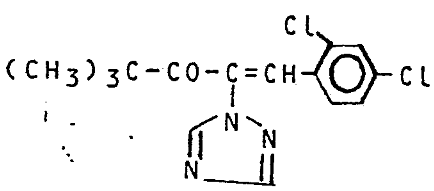

(E) 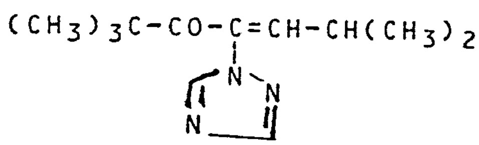

(F) 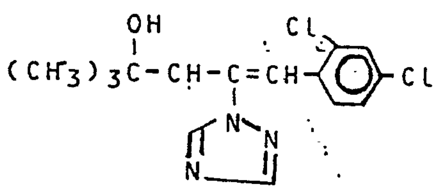

(G) 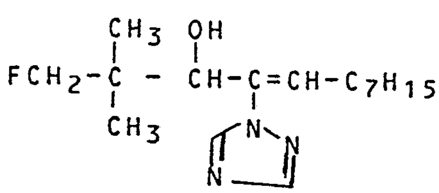

(H) 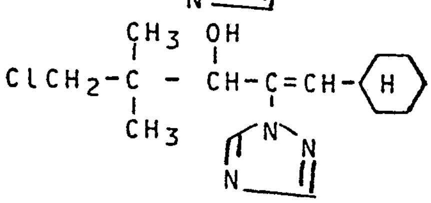

**Beispiel A**

Venturia-Test (Apfel) / protektiv/
Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 50, 53, 2, 55, 33, 3, 62, 63 und 69.

**Beispiel B**

Erysiphe-Test (Gerste) / protektiv

Lösungsmittel 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgestellt, un die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 18, 11, 10, 17, 16, 7, 6, 21, 20, 38, 37, 36, 1, 27, 28, 29, 33, 3, 34, 77, 81, 79, 82, 87, 85, 83, 75, 74, 46, 49, 42, 39, 65, 55, 93, 97, 96, 94, 88, 91, 90, 89 und 92.

**Beispiel C**

Puccinia-Test (Weizen) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 14, 51, 50, 43, 45, 53, 2, 54.

**Beispiel D**

Cochliobolus sativus-Test (Gerste) /protektiv

Lösungsmittel: 100 Gewichtsteile Dimethylformamid

Emulgator: 0,25 Gewichtsteile Alkylarylpolyglykolether

Zu Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung

taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20° und einer relativen Luftfeuchtigkeit von ca. 80 % aufgestellt.

7 Tage nach der Inokulation erfolgt die Auswertung.

Eine deutliche Ueberlegeheit in der Wirksamkeit gegenüber dem Stand der Technik zeigen bei diesem Test z.B. die Verbindungen gemäß folgender Herstellungsbeispiele: 24, 53, 2, 54, 55, 85, 74 und 88.

**Beispiel E**

**Wuchsbeeinflussung bei Zuckerrüben**

Lösungsmittel: 30 Gewichtsteile Dimethylformamid

Emulgator: 1 Gewichtsteile Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive eine Wuchsförderung gegenüber den Kontrollpflanzen.

Die erfindungsgemäßen Wirkstoffe 49, 96, 39, 88, 91, 90, 89, 65, 66, 24, 53, 2, 54, 55, 56, 67 und 71 zeigen bei diesem Test starke Wuchsbeeinflussung.

**Beispiel F**

**Wuchshemmung bei Baumwolle**

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf. Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrolle berechnet. Es bedeuten 100 % Wuchshemmung den stillstand des Wachstums und 0 % ein Wachstum entsprechend dem der Kontrollpflanzen.

Die erfindungsggemäßen Wirkstoffe 19, 9, 96, 39, 90, 21, 66, 2, 55, 67 und 70 zeigen bei diesem Test eine starke Wuchshemmung.

**Beispiel G**

**Stimulierung der $CO_2$-Fixierung bei Sojabohnen**

Lösungsmittel: 30 Gewichtateile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Forlgeblattes angezogen. In diesem Stadium werden die Pflanzen tropfnaß mit den Wirkstoffzubereitungen besprüht. Im weiteren Verlauf des Experimentes wird die $CO_2$-Fixierung der Pflanzen mit üblichen Methoden gemessen. Die Werte werden mit denen nicht mit Wirkstoffen behandelter Kontrollpflanzen vergleichen.

Die erfindungsgemäßen Wirkstoffe 23, 7, 6, 21 und 36 zeigen gute Stimulierung der $CO_2$-Fixierung.

**Beispiel H**

**Wuchshemmung bei Sojabohnen**

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 5 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die erfindungsgemäßen Wirkstoffe 90, 58, 67, 70 und 71 zeigen bei diesem Test starke Wuchshemmung.

**Patentansprüche**

1. Substituierte Azolylvinyl-ketone und -carbinole der Formel

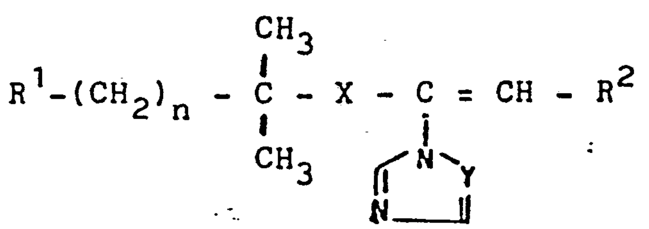

(I)

in welcher

$R^1$ für gegebenenfalls einfach bis dreifach, gleich oder verschieden substituiertes Phenyl steht, wobei als Substituenten Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino und gegebenenfalls durch Fluor, Chlor und Methyl substituiertes Phenyl oder Phenoxy zu nennen sind; ferner für Vinyl, Propargyl, Cyano, sowie die Gruppierung -Z-$R^3$ steht;

$R^3$ für jeweils gegebenenfalls einfach bis dreifach, gleich oder verschieden substituiertes Phenyl und Benzyl steht, wobei jeweils als Phenylsubstituenten die bei $R^1$ bereits oben genannten Phenylsubstituenten infrage kommen;

Z für O, S, SO oder $SO_2$ steht,

n für die Zahlen 0 bis 2 steht,

$R^2$ für geradkettiges oder verzweigtes Alkyl mit 1 bis 8 Kohlenstoffatomen und geradkettiges oder verzweigtes Alkenyl und Alkinyl mit jeweils 2 bis 4 Kohlenstoffatomen steht; ferner für jeweils gegebenenfalls einfach bis dreifach, gleich oder verschieden substituiertes Phenyl und Phenylalkyl mit 1 bis 2 Kohlenstoffatomen im Alkylteil steht, wobei jeweils als Phenylsubstituenten die bei $R^1$ bereits genannten Phenylsubstituenten infrage kommen: weiterhin für gegebenenfalls einfach bis dreifach, gleich oder verschieden durch Methyl, Ethyl, Isopropyl, Fluor oder Chlor substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cycloheptylmethyl, Cycloalkenyl mit 3 bis 7 Kohlenstoffatomen, Cycloalkenylmethyl mit 3 bis 7 Kohlenstoffatomen im Cycloalkenylteil, Bicycloalkyl mit 5 bis 12 Kohlenstoffatomen oder Bicycloalkenyl mit 5 bis 12 Kohlenstoffatomen steht; wobei jedoch nicht gleichzeitig $R^2$ für gegebenenfalls substituiertes Phenyl und $R^1$ für gegebenenfalls substituiertes Phenyl stehen dürfen;

X für die CO- oder CH(OH)-Gruppe steht und

Y für ein Stickstoffatom oder die CH-Gruppe steht,

sowie deren Säureadditions-Salze und Metallsalz-Komplexe.

2. Verfahren zur Herstellung von substituierten Azolylvinyl-ketonen und -carbinolen der Formel

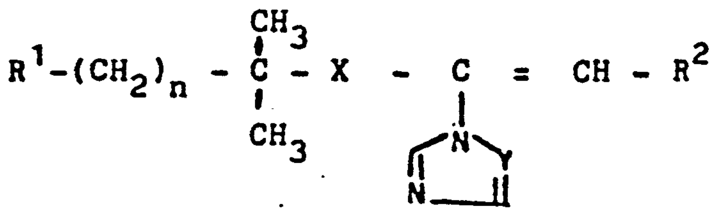

(I)

in welcher

$R^1$ für gegebenenfalls einfach bis dreifach, gleich oder verschieden substituiertes Phenyl steht, wobei als Substituenten Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino und gegebenenfalls durch Fluor, Chlor und Methyl substituiertes Phenyl oder Phenoxy zu nennen sind; ferner für Vinyl, Propargyl, Cyano, sowie die Gruppierung -Z-$R^3$ steht;

$R^3$ für jeweils gegebenenfalls einfach bis dreifach, gleich oder verschieden substituiertes Phenyl und Benzyl steht, wobei jeweils als Phenylsubstituenten die bei $R^1$ bereits oben genannten Phenylsubstituenten infrage kommen;

Z für O, S, SO oder $SO_2$ steht,

n für die Zahlen 0 bis 2 steht,

$R^2$ für geradkettiges oder Verzweigtes Alkyl mit 1 bis 8 Kohlenstoffatomen und geradkettiges oder verzweigtes Alkenyl und Alkinyl mit jeweils 2 bis 4 Kohlenstoffatomen steht; ferner für jeweils gegebenenfalls einfach bis dreifach, gleich oder verschieden substituiertes Phenyl und Phenylalkyl mit 1 bis 2 Kohlenstoffatomen im Alkylteil steht, wobei jeweils als Phenylsubstituenten die bei $R^1$ bereits genannten Phenylsubstituenten infrage kommen: weiterhin für gegebenenfalls einfach bis dreifach, gleich oder verschieden durch Methyl, Ethyl, Isopropyl, Fluor oder Chlor substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cycloheptylmethyl, Cycloalkenyl mit 3 bis 7 Kohlenstoffatomen, Cycloalkenylmethyl mit 3 bis 7 Kohlenstoffatomen im Cycloalkenylteil, Bicycloalkyl mit 5 bis 12 Kohlenstoffatomen oder Bicycloalkenyl mit 5 bis 12 Kohlenstoffatomen steht; wobei jedoch nicht gleichzeitig $R^2$ für gegebenenfalls substituiertes Phenyl und $R^1$ für gegebenenfalls substituiertes Phenyl stehen dürfen;

X für die CO- oder CH(OH)- Gruppe steht und

Y für ein Stickstoffatom oder die CH-Gruppe steht,

sowie von deren Säureadditions-Salzen und Metallsalz-Komplexen,

dadurch gekennzeichnet, daß man a) Ketoenamine der Formel

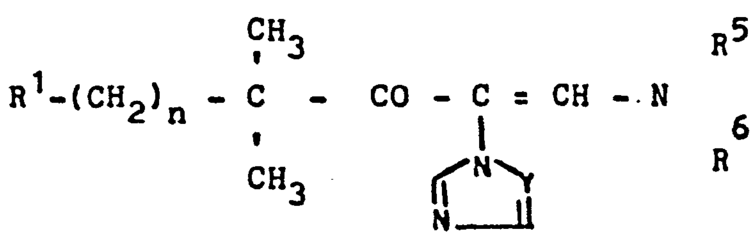

(II)

in welcher

$R^1$, Y und n die oben angegebene Bedeutung haben und

$R^5$ und$R^6$ gleich oder verschieden sind und für Alkyl mit 1 bis 4 Kohlenstoffatomen stehen; oder gemeinsam mit dem N-Atom, an das sie gebunden sind, für jeweils gegebenenfalls einfach oder dreifach durch Alkyl mit 1 bis 4 Kohlenstoffatomen substituiertes Piperidinyl, Pyrrolidinyl und Morpholinyl stehen,

mit magnesium-organischen Verbindungen der Formel

Hal- Mg- $R^2$ (III)

in welcher

$R^2$ - die oben angegebene Bedeutung hat und

Hal für Halogen steht,

in Gegenwart eines Lösungsmittels und gegebenenfalls in Gegenwart eines Inertgases umsetzt, oder

b) Azolylketone der Formel

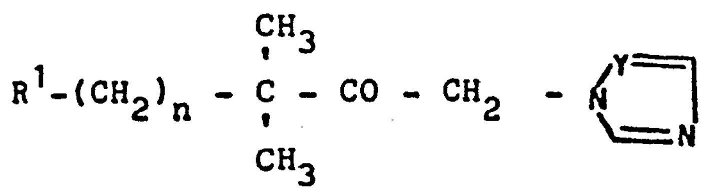

(IV)

in welcher
$R^1$, Y und n die Oben angegebene Bedeutung haben,
mit Aldehyden der Formel
$O = CH - R^2$ (V)
in welcher
$R^2$ die oben angegebene Bedeutung hat,
in Gegenwart eines Lösungsmittels und in Gegenwart eines Katalysators umsetzt, und

c) gegebenenfalls die nach dem Verfahren (a) und (b) erhaltenen erfindungsgemäßen substituierten Azolylvinyl-ketone der Formel

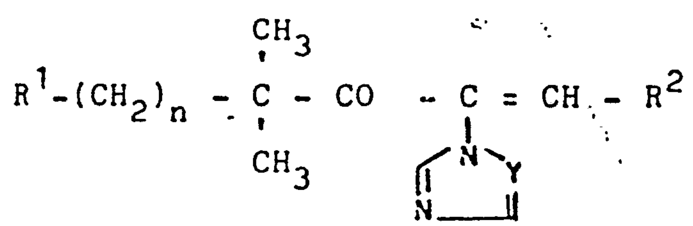

(Ia)

$R^1$, $R^2$, Y und n die oben angegebene Bedeutung haben,
in allgemein üblicher Weise reduziert,
und gegebenenfalls an die so erhaltenen Verbindungen der Formel (1) noch eine Säure oder ein Metallsalz addiert.

3. Pflanzenschutzmittel, gekennzeichnet durch einen Gehalt an mindestens einen substituierten Azolylvinylketon oder -carbinol der Formel (I) in Anspruch 1 bzw. an einem Säureadditions-Salz oder Metallsalz-Komplex einer Verbindung oder Formel (I).

4. Fungizide und das Pflanzenwachstum regulierende Mittel, gekennzeichnet durch einen Gehalt an mindestens einem substituierten Azolylvinyl-keton oder -carbinol der Formel (I) in Anspruch 1 bzw. an einem Säureadditions-Salz oder Metallsalz-Komplex einer Verbindung der Formel (I).

5. Verfahren zur Bekämpfung von Pilzen und zur Regulierung des Pflanzenwachstums, dadurch gekennzeichnet, daß man substituierte Azolylvinyl-ketone oder -carbinole der Formel (I) in Anspruch 1 bzw. deren Säureadditions-Salze oder Metallsalz-Komplexe auf Pilze, Pflanzen oder ihren Lebensraum einwirken läßt.

6. Verwendung von substituierten Azolylvinyl-ketonen oder-carbinolen der Formel (I) in Anspruch 1 bzw. von deren Säureadditions-Salzen oder Metallsalz-Komplexen als Pflanzenschutzmittel.

7. Verwendung von substituierten Azolylvinyl-ketonen oder -carbinolen der Formel (I) in Anspruch 1 bzw deren Säureadditions-Salzen oder Metallsalz-Komplexen zur Bekämpfung von Pilzen und zur Regulierung des Pflanzenwachstums.

**Claims**

1. Substituted azolylvinyl ketones and carbinols of the formula

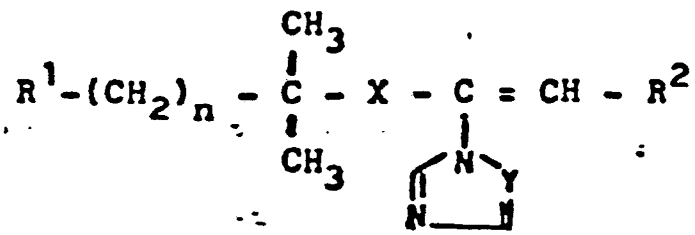

(I)

in which

$R^1$ represents phenyl which is optionally mono- to trisubstituted by identical or different substituents, substituents which may be mentioned being fluorine, chlorine, methyl, isopropyl, tert.-butyl, methoxy, methylthio, isopropoxy, trifluoromethyl, trifluoromethoxy, trifluoromethylthio, methylamino, dimethylamino and phenoxy or phenyl optionally substituted by fluorine, chlorine and methyl; and furthermore represents vinyl, propargyl, cyano and the grouping $-Z-R^3$;

$R^3$ represents benzyl and phenyl, each of which is optionally mono- to trisubstituted by identical or different substituents, possible phenyl substituents in each case being the phenyl substituents already mentioned above under $R^1$;

Z represents O, S, SO or $SO_2$,

n represents the numbers 0 to 2,

$R^2$ represents straight-chain or branched alkyl with 1 to 8 carbon atoms and straight-chain or branched alkenyl and alkinyl each with 2 to 4 carbon atoms;

furthermore represents phenylalkyl with 1 to 2 carbon atoms in the alkyl part and phenyl, each of which isoptionally mono- to trisubstituted by identical or different substituents, possible phenyl substituents in each case being the phenyl substituents already mentioned under $R^1$; and furthermore represents cyclopropyl, cyclobutyl, cyclopentyl, cyclohexyl, cyclopropylmethyl, cyclobutylmethyl, cyclopentylmethyl, cyclohexylmethyl, cycloheptylmethyl, cycloalkenyl with 3 to 7 carbon atoms, cycloalkenylmethyl with 3 to 7 carbon atoms in the cycloalkenyl part, bicycloalkyl with 5 to 12 carbon atoms or bicycloalkenyl with 5 to 12 carbon atoms, each of which is optionally mono- to trisubstituted by identical or different substituents from the group comprising methyl, ethyl, isopropyl, fluorine or chlorine; but $R^2$ may not represent optionally substituted phenyl at the same time as $R^1$ represents optionally substituted phenyl;

X represents the CO or CH(OH) group and

Y represents a nitrogen atom or the CH group, and acid addition salts and metal salt complexes thereof.

2. Process for the preparation of substituted azolylvinyl ketones and carbinols of the formula

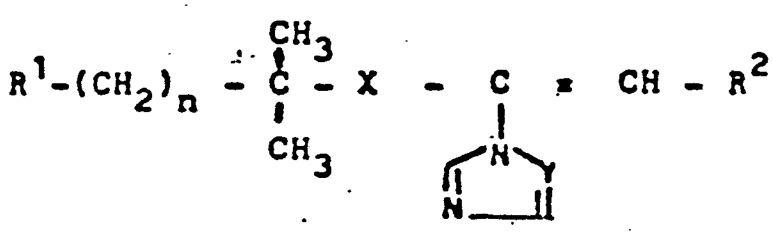

(I)

in which

$R^1$ represents phenyl which is optionally mono- to trisubstituted by identical or different substituents, substituents which may be mentioned being fluorine, chlorine, methyl, isopropyl, tert.-butyl, methoxy, methylthio, isopropoxy, trifluoromethyl, trifluoromethoxy, trifluoromethylthio, methylamino, dimethylamino and phenoxy or phenyl optionally substituted by fluorine, chlorine and methyl; and furthermore represents

vinyl, propargyl, cyano and the grouping -Z-$R^3$;,

$R^3$ represents benzyl and phenyl, each of which is optionally mono- to trisubstituted by identical or different substituents, possible phenyl substituents in each case being the phenyl substituents already mentioned above under $R^1$;,

Z represents O, S, SO or $SO_2$

n represents the numbers 0 to 2,

$R^2$ represents straight-chain or branched alkyl with 1 to 8 carbon atoms and straight-chain or branched alkenyl and alkinyl each with 2 to 4 carbon atoms; furthermore represents phenylalkyl with 1 to 2 carbon atoms in the alkyl part and phenyl, each of which is optionally mono- to trisubstituted by identical or different substituents, possible phenyl substituents in each case being the phenyl substituents already mentioned under $R^1$; and furthermore represents cyclopropyl, cyclobutyl, cyclopentyl, cyclohexyl, cyclopropylmethyl, cyclobutylmethyl, cyclopentylmethyl, cyclohexylmethyl, cycloheptylmethyl, cycloalkenyl with 3 to 7 carbon atoms, cycloalkenylmethyl with 3 to 7 carbon atoms in the cycloalkenyl part, bicycloalkyl with 5 to 12 carbon atoms or bicycloalkenyl with 5 to 12 carbon atoms, each of which is optionally monoto trisubstituted by identical or different substituents from the group comprising methyl, ethyl, isopropyl, fluorine or chlorine; but $R^2$ may not represent optionally substituted phenyl at the same time as $R^1$ represents optionally substituted phenyl;

X represents the CO or CH(OH) group and

Y represents a nitrogen atom or the CH group, and acid addition salts and metal salt complexes thereof, characterised in that

a) ketoenamines of the formula

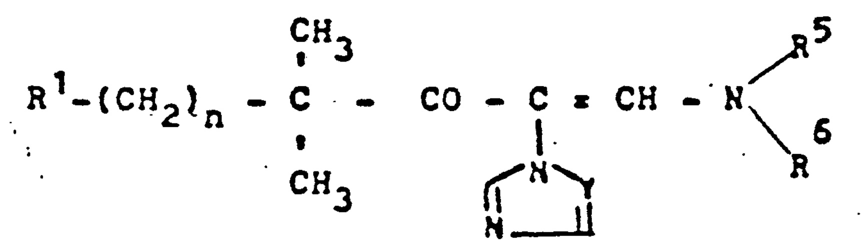

(II)

in which

$R^1$, Y and n have the above mentioned meaning and

$R^5$ and $R^6$ are identical or different and represent alkyl with 1 to 4 carbon atoms; or, together with the N atom to which they are bonded, represent piperidinyl, pyrrolidinyl and morpholinyl, each of which is optionally mono- or trisubstituted by alkyl with 1 to 4 carbon atoms, are reacted with organomagnesium compounds of the formula

Hal- Mg- $R^2$ (III)

in which

$R^2$ has the above mentioned meaning and

Hal represents halogen,

in the presence of a solvent and if appropriate in the presence of an inert gas, or

b) azolyl ketones of the formula

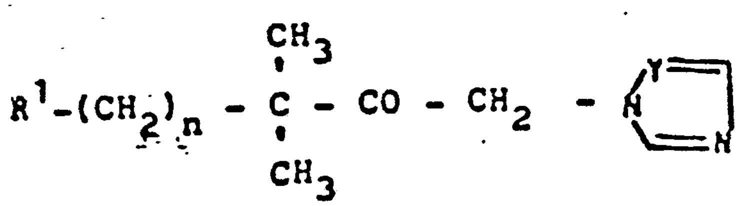

(IV)

in which
$R^1$, Y and n have the above mentioned meaning, are reacted with aldehydes of the formula
$O = CH - R^2$ (V)
in which
$R^2$ has the above mentioned meaning,
in the presence of a solvent and in the presence of a catalyst, and
c) if desired, the azolylvinyl ketones according to the invention of the formula

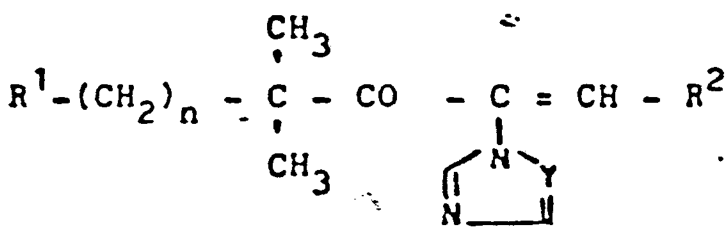

(Ia)

$R^1$, $R^2$, Y and n have the above mentioned meaning obtained by processes (a) and (b) are reduced in a generally customary manner, and, if desired, an acid or a metal salt is then added on to the compounds of the formula (I) thus obtained.

3. Plant protection agents, characterised in that they contain at least one substituted azolylvinyl ketone or carbinol of the formula (I) in Claim 1 or an acid addition salt or metal salt complex of a compound or formula (I).

4. Fungicidal agents and agents regulating plant growth, characterised in that they contain at least one substituted azolylvinyl ketone or carbinol of the formula (I) in Claim 1 or an acid addition salt or metal salt complex of a compound of the formula (I).

5. Process for combating fungi and for regulating plant growth, characterised in that substituted azolylvinyl ketones or carbinols of the formula (I) in Claim 1 or acid addition salts or metal salt complexes thereof are allowed to act on fungi, plants or their environment.

6. Use of substituted azolylvinyl ketones or carbinols of the formula (I) in Claim 1 or acid addition salts or metal salt complexes thereof as plant protection agents.

7. Use of substituted azolylvinyl ketones or carbinols of the formula (I) in Claim 1 or acid addition salts or metal salt complexes thereof for combating fungi and for regulating plant growth.

**Revendications**

1. Azolylvinyl-cétones et -carbinols substitués de formule:

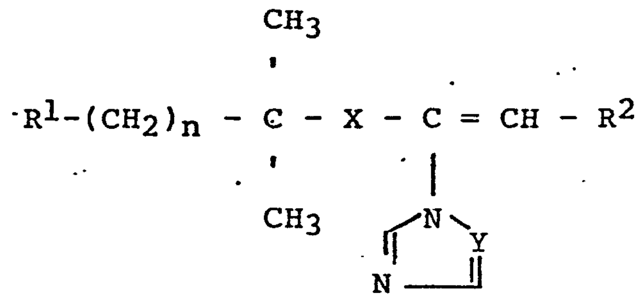 (I)

dans laquelle

$R^1$ représente un phényle éventuellement substitué une à trois fois, de manière identique ou différente, en citant comme substituants le fluor, chlore, méthyle, isopropyle, t-butyle, méthoxy, méthylthio, isopropoxy, trifluorométhyle, trifluorométhoxy, trifluorométhylthio, méthylamino, diméthylamino et éventuellement un phényle ou phénoxy éventuellement substitués par du fluor, du chlore et un méthyle, en outre vinyle, propargyle, cyano de même que le groupement $-Z-R^3$,

$R^3$ représente un phényle et benzyle éventuellement substitués chacun une à trois fois, de manière identique ou différente, en envisageant chaque fois comme substituants du phényle les substituants du phényle déjà cités plus haut pour $R^1$,

Z représente O, S, SO ou $SO_2$,

n représente les nombres 0 à 2,

$R^2$ représente un alcoyle à chaîne droite ou ramifiée ayant 1 à 8 atomes de carbone et un alcényle et alcinyle à chaîne droite ou ramifiée ayant chacun 2 à 4 atomes de carbone, en outre un phényle et phénylalcoyle éventuellement substitués chacun une à trois fois, de manière identique ou différente et ayant 1 à 2 atomes de carbone dans la portion alcoyle, en envisageant chaque fois comme substituants du phényle les substituants du phényle déjà cités pour $R^1$, de plus un cyclopropyle, cyclobutyle, cyclopentyle, cyclohexyle, cyclopropylméthyle, cyclobutylméthyle, cyclopentylméthyle, cyclohexylméthyle, cycloheptylméthyle, cycloalcényle ayant 3 à 7 atomes de carbone, cycloalcénylméthyle ayant 3 à 7 atomes de carbone dans la portion cycloalcényle, bicycloalcoyle ayant 5 à 12 atomes de carbone ou bicycloalcényle ayant 5 à 12 atomes de carbone, lesquels sont éventuellement substitués une à trois fois, de manière identique ou différente par un méthyle, éthyle, isopropyle, fluor ou chlore, avec la restriction que $R^2$ ne peut pas représenter un phényle éventuellement substitué en même temps que $R^1$ représente un phényle éventuellement substitué,

X représente le groupe CO ou CH(OH) et

Y représente un atome d'azote ou le groupe CH, ainsi que leurs sels d'addition d'acides et complexes avec un sel métallique.

2. Procédé de fabrication d'azolylvinylcétones et -carbinols substitués de formule:

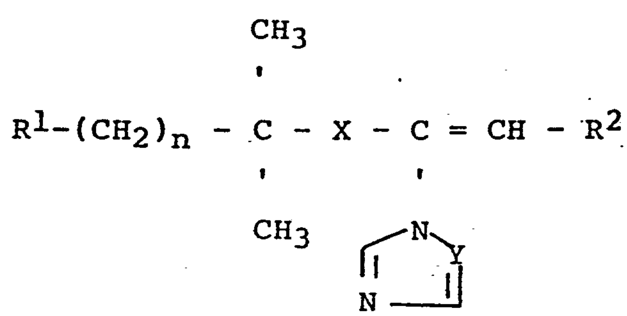 (I)

dans laquelle

$R^1$ représente un phényle éventuellement substitué une à trois fois, de manière identique ou différente, en citant comme substituants le fluor, chlore, méthyle, isopropyle, t-butyle, méthoxy, méthylthio, isopropoxy, trifluorométhyle, trifluorométhoxy, trifluorométhylthio, méthylamino, diméthylamino et éventuellement un phényle ou phénoxy éventuellement substitués par du fluor, du chlore et un méthyle, en outre vinyle, propargyle, cyano de même que le groupement $-Z-R^3$,

$R^3$ représente un phényle et benzyle éventuellement substitués chacun une à trois fois, de manière identique

ou différente, en envisageant chaque fois comme substituants du phényle les substituants du phényle déjà cités plus haut pour $R^1$,

Z représente O, S, SO ou $SO_2$,

n représente les nombres 0 à 2,

$R^2$ représente un alcoyle à chaîne droite ou ramifiée ayant 1 à 8 atomes de carbone et un alcényle et alcinyle à chaîne droite ou ramifiée ayant chacun 2 à 4 atomes de carbone, en outre un phényle et phénylalcoyle éventuellement substitués chacun une à trois fois, de manière identique ou différente et ayant 1 à 2 atomes de carbone dans la portion alcoyle, en envisageant chaque fois comme substituants du phényle les substituants du phényle déjà cités pour $R^1$, de plus un cyclopropyle, cyclobutyle, cyclopentyle, cyclohexyle, cyclopropylméthyle, cyclobutylméthyle, cyclopentylméthyle, cyclohexylméthyle, cycloheptylméthyle, cycloalcényle ayant 3 à 7 atomes de carbone, cycloalcénylméthyle ayant 3 à 7 atomes de carbone dans la portion cycloalcényle, bicycloalcoyle ayant 5 à 12 atomes de carbone ou bicycloalcényle ayant 5 à 12 atomes de carbone, lesquels sont éventuellement substitués une à trois fois, de manière identique ou différente par un méthyle, éthyle, isopropyle, fluor ou chlore, avec la restriction que $R^2$ ne peut pas représenter un phényle éventuellement substitué en même temps que $R^1$ représente un phényle éventuellement substitué,

X représente le groupe CO ou CH(OH) et

Y représente un atome d'azote ou le groupe CH, ainsi que leurs sels d'addition d'acides et complexes avec un sel métallique, caractérisé en ce que

a) on fait réagir des céto-ènamines de formule:

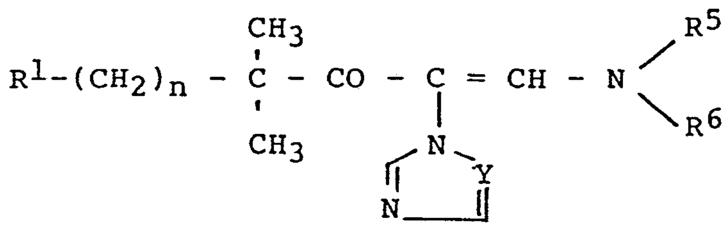

(II)

dans laquelle

$R^1$, Y et n ont la signification indiquée plus haut et

$R^5$ et $R^6$ sont identiques ou différents et représentent un alcoyle ayant 1 à 4-atomes de carbone, ou bien, en commun avec l'atome N sur lequel ils sont fixés, représentent un pipéridinyle, pyrrolidinyle et morpholinyle éventuellement substitués chacun une à trois fois par un alcoyle ayant 1 à 4 atomes de carbone,

avec des composés organo-magnésiens de formule:

Hal - Mg - $R^2$ (III)

dans laquelle

$R^2$ a la signification indiquée plus haut et

Hal représente de l'halogène,

en présence d'un solvant et éventuellement en présence d'un gaz inerte, ou

b) on fait réagir des azolylcétones de formule:

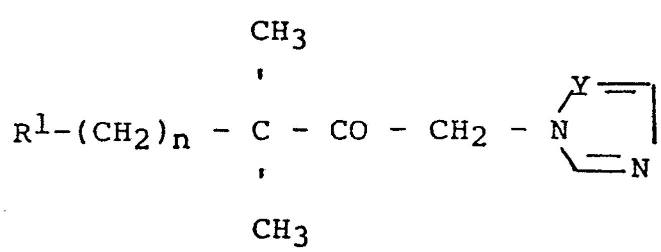

(IV)

dans laquelle

$R^1$, Y et n ont la signification indiquée plus haut,

avec des aldéhydes de formule:
O = CH - $R^2$ (V)
dans laquelle
$R^2$ a la signification indiquée plus haut, en présence d'un solvant et en présence d'un catalyseur, et

c) le cas échéant on réduit de la manière en usage général les azolylvinylcétones substituées conformes à l'invention obtenues par les procédés (a) et (b), de formule:

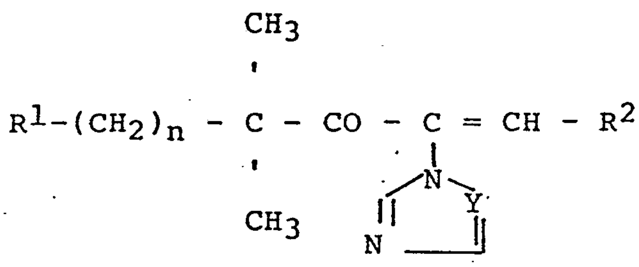

(Ia)

$R^1$, $R^2$, Y et n ayant les significations indiquées plus haut, et sur les composés ainsi obtenus de formule (I) on fixe éventuellement encore un acide ou un sel métallique.

3. Agents de protection des plantes, caractérisés par une teneur en au moins un azolylvinylcétone ou -carbinol substitué de formule (I) donnée dans la revendication 1 ou en un sel d'addition d'acide ou complexe avec un sel métallique d'un composé de formule (I).

4. Agents fongicides et régulateurs de la croissance des plantes, caractérisés par une teneur en au moins un azolylvinyl-cétone ou -carbinol substitué de formule (I) donnée dans la revendication 1 ou en un sel d'addition d'acide ou complexe avec un sel métallique d'un composé de formule (I).

5. Procédé pour combattre les champignons et pour régler la croissance des plantes, caractérisé en ce qu'on fait agir des azolylvinyl-cétones ou -carbinols substitués de formule (I) donnée dans la revendication 1 ou leurs sels d'addition d'acides ou complexes avec un sel métallique sur des champignons, sur des plantes ou sur leur espace vital.

6. Utilisation d'azolylvinyl-cetones ou -carbinols substitués de formule (I) donnée dans la revendication 1 ou de leurs sels d'addition d'acides ou complexes avec un sel métallique en tant qu'agents de protection des plantes.

7. Utilisation d'azolylvinyl-cétones ou -carbinols substitués de formule (I) donnée dans la revendication 1 ou de leurs sels d'addition d'acides ou complexes avec un sel métallique pour combattre les champignons et pour régler la croissance des plantes.